# FASCICULE DE BREVET EUROPEEN

(11) **EP 0 722 928 B1**
(45) Date de publication et mention de la délivrance du brevet: **06.08.1997**
(21) Numéro de dépôt: 95120073.2
(22) Date de dépôt: 19.12.1995
(51) Int. Cl.: C07C 65/26, A61K 31/19, C07C 59/72

(54) **Composés bicycliques-aromatiques à forte activité biologique, compositions pharmaceutiques et cosmétiques les contenant et utilisations**
Bicyclische aromatische Verbindungen mit starker biologischer Wirksamkeit, pharmazeutische und kosmetische Zusammensetzungen mit diesen Verbindungen sowie ihre Verwendung
Bicyclic-aromatic compounds with strong biologic activity, pharmaceutical and cosmetic compositions containing them and uses thereof

(30) Priorité: 20.01.1995 FR 9500659
(43) Date de publication de la demande: 24.07.1996
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, F-06650 le Rouret (FR)
(74) Mandataire: Tezier Herman, Béatrice

(56) Documents cités:
- US-A- 5 248 823

## Description

L'invention concerne, à titre de produits industriels nouveaux et utiles, des composés bicycliques-aromatiques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 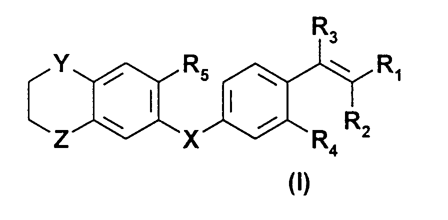 dans laquelle:
* R₁ représente
   (i) un atome d'hydrogène
   (ii) le radical -CH₃
   (iii) le radical -CH₂OR₆
   (iv) le radical -O-R₈,uniquement lorsque R₂ et R₄ forment avec le cycle benzénique adjacent un cycle naphthalénique;
   (v) le radical -CO-R₁₀
   (vi) un radical -S(O)p-R₁₂
   R₆, R₈, R₁₀, R₁₂ et p ayant les significations données ci-après,
* R2 et R3 sont soit indépendants, soit pris ensemble,
   - dans le cas où ils sont indépendants :
      R₂ représente un atome d'hydrogène, un radical alkyle inférieur, un radical hydroxy,un radical -OR₇, un radical -O-COR₇, un radical amino ou un radical -NH-COR₇;
      R3 représente un atome d'hydrogène ou un radical alkyle inférieur;
   - dans le cas où ils sont pris ensemble :
      R₂ et R3 forment avec la liaison éthylénique une liaison acétylénique;
* R₂ et R₄ sont soit indépendants, soit pris ensemble,
   - dans le cas où ils sont indépendants :
      R₂ a la signification donnée ci-dessus;
      R₄ représente un atome d'hydrogène, un radical alkyle linéaire ou ramifié ayant de 1 à 20 atomes de carbone, un radical hydroxy, un radical - OR₇, un radical -O-COR₇;
   - dans le cas où ils sont pris ensemble :
      R₂ et R₄ forment avec le cycle benzénique adjacent un cycle naphthalénique.
      R₇ ayant la signification donnée ci-après;
* R₅ représente un atome d'hydrogène, un atome d'halogène, un radical alkyle inférieur, un radical NO₂, un radical hydroxy, un radical -OR₇, un radical - O-COR₇, ou bien encore un radical 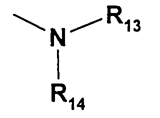 R₇, R₁₃ et R₁₄ ayant les significations données ci-après;
* X représente -O-, -S(O)ₜ- ou -NR₁₂-,
   t et R₁₂ ayant les significations données ci-après ;
* Y et Z, identiques ou différents, représentent -CR₁₃R₁₄-, -O-, -S(O)ₜ-, avec la condition que Y et Z ne sont pas simultanément un atome d'oxygène ou simultanément un radical -S(O)ₜ ;
   t, R₁₃ et R₁₄ ayant les significations données ci-après;
étant entendu que dans tout ce qui précède :
R₆ représente un atome d'hydrogène, un radical alkyle inférieur, un radical -COR₇ ;
R₇ représente un radical alkyle inférieur
R₈ représente un atome d'hydrogène, un radical alkyle inférieur, un radical -(CH2)m-(CO)n-R₉
R₉ représente un radical alkyle inférieur ou un hétérocycle
R₁₀ représente:
   (a) un atome d'hydrogène;
   (b) un radical alkyle inférieur;
   (c) un radical 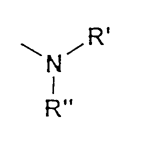
   (d) un radical-O-R₁₁
R₁₁ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre ou un reste d'amino acide ou de peptide;
R₁₂ représente un atome d'hydrogène, un radical alkyle inférieur;
R₁₃ et R₁₄ représentent un atome d'hydrogène, un radical alkyle inférieur;
m est un entier variant de 1 à 3;
n est 0 ou 1;
p est 0 ou un entier variant de 1 à 3;
t est 0, 1 ou 2;
R' et R'' représentent indépendamment un atome d'hydrogène, un radical alkyle inférieur, un radical mono ou polyhydroxyalkyle, un radical aryle éventuellement substitué ou un reste d'amino acide ou de sucre ou encore pris ensemble forment un hétérocycle.

L'invention vise également les sels des composés de formule (I) ci-dessus dans le cas où R₁ représente une fonction acide carboxylique, une fonction acide sulfonique ou encore lorsque le radical R₅ représente une fonction amine. Elle vise aussi les isomères optiques et géométriques desdits composés.

Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 12, de préférence de 1 à 9, atomes de carbone, avantageusement les radicaux méthyle, éthyle, isopropyle, butyle, tertiobutyle, hexyle, nonyle et dodécyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les atomes d'halogène, on préfère l'atome de fluor, de chlore ou de brome.

Parmi les composés de formule (I) ci-dessus rentrant dans le cadre de la présente invention, on peut notamment citer les suivants :
Acide 6-(5,6,7,8-tétrahydro-5,5,8,8-tétraméthyl-2-naphtyloxy)naphtoïque
Acide 4-(5,6,7,8-tétrahydro-5,5,8,8-tétraméthyl-2-naphtyloxy)cinnamique
Acide β-methyl-4-(5,6,7,8-tétrahydro-5,5,8,8-tétraméthyl-2-naphtyloxy)cinnamique
Acide 4-(5,6,7,8-tétrahydro-5,5,8,8-tétraméthyl-2-naphtyloxy)phenylpropiolique
Acide 6-(3,5,5,8,8-pentaméthyl-5,6,7,8-tétrahydro-2-naphtyloxy)naphtoïque
Acide 6-(5,6,7,8-tétrahydro-5,5,8,8-tétraméthyl-2-naphtylthio)naphtoïque
Acide 6-(5,6,7,8-tétrahydro-5,5,8,8-tétraméthyl-2-naphtylsulfinyl)naphtoïque
Acide 6-(5,6,7,8-tétrahydro-5,5,8,8-tétraméthyl-2-naphtylsulfonyl)naphtoïque
Acide 6-(5,6,7,8-tétrahydro-5,5,8,8-tétraméthyl-2-naphtylamino)naphtoïque
Acide α-hydroxy-4-(5,6,7,8-tétrahydro-5,5,8,8-tétraméthyl-2-naphtyloxy)cinnamique
Acide α-acetamido-4-(5,6,7,8-tétrahydro-5,5,8,8-tétraméthyl-2-naphtyloxy)cinnamique
Acide 6-(3,5,5,8,8-pentaméthyl-5,6,7,8-tétrahydro-2-naphtylthio)naphtoïque
Acide 6-(3,5,5,8,8-pentaméthyl-5,6,7,8-tétrahydro-2-naphtylsulfonyl)naphtoïque
Acide 6-(3-bromo-5,6,7,8-tétrahydro-5,5,8,8-tétraméthyl-2-naphtyloxy)naphtoïque
6-(5,6,7,8-tétrahydro-5,5,8,8-tétraméthyl-2-naphtyloxy)naphtalène méthanol
N-éthyl-6-(5,6,7,8-tétrahydro-5,5,8,8-tétraméthyl-2-naphtyloxy)naphtalène carboxamide
6-(5,6,7,8-tétrahydro-5,5,8,8-tétraméthyl-2-naphtyloxy)naphtalène carboxamide
Morpholide de l'acide 6-(5,6,7,8-tétrahydro-5,5,8,8-tétraméthyl-2-naphtyloxy)naphtoïque
6-(5,6,7,8-tétrahydro-5,5,8,8-tétraméthyl-2-naphtyloxy)naphtalène carboxaldéhyde

Selon la présente invention, les composés de formule (I) préférés sont ceux pour lesquels l'une au moins des conditions ci-dessous sont respectées :
- R₁ est un radical -CO-R₁₀ ;
- X représente -O-, -S-, -NR₁₂- ;
- R₂ représente un atome d'hydrogène ou un radical alkyle inférieur ;
- R₂ et R₄ forment avec le cycle benzénique adjacent un cycle naphtalénique ;
- R₂ et R₃ forment avec la liaison éthylénique une liaison acétylénique.

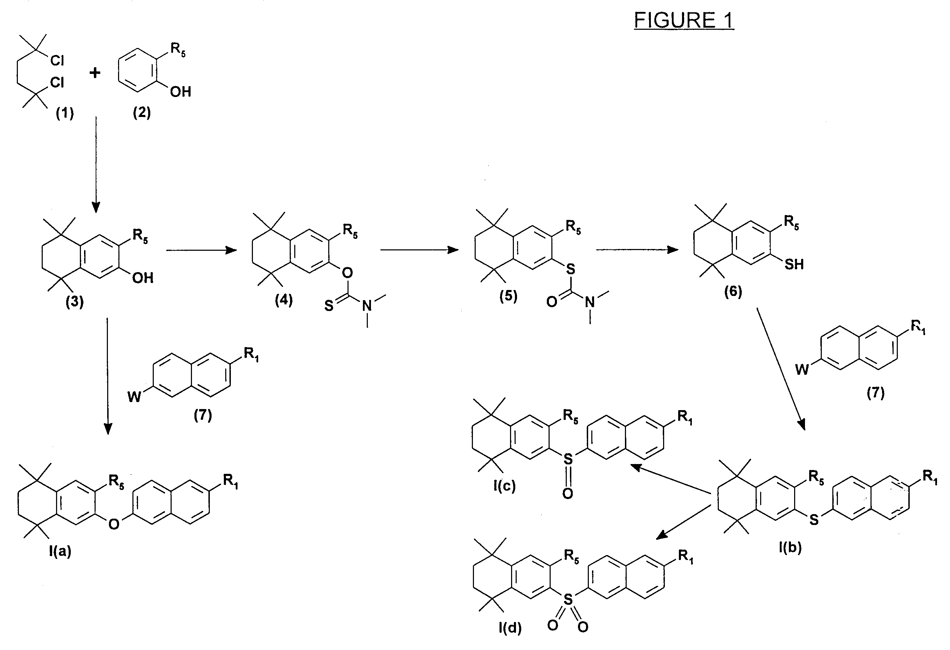

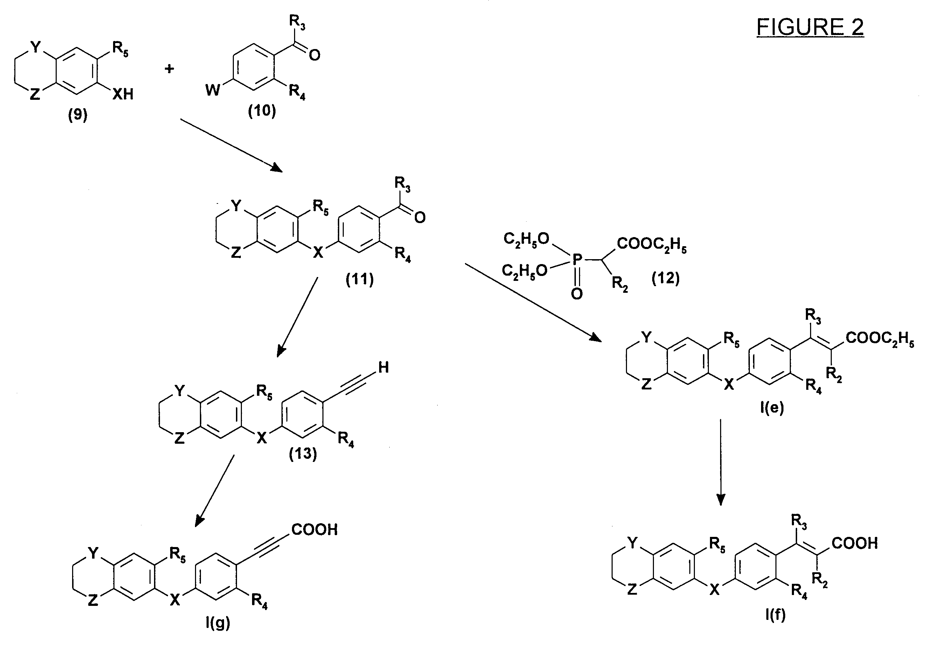

La présente invention a également pour objet les procédés de préparations des composés de formule (I), en particulier selon les schémas réactionnels donnés aux figures 1 et 2.

Ainsi, les composés de formule I (a) peuvent être obtenus (figure 1) à partir du sel de sodium du dérivé phénolique (3) par couplage avec un dérivé halogéné (7), de préférence un dérivé bromé ou iodé, en présence d'un complexe de bromure de cuivre et de diméthyl sulfure dans un solvant tel que la pyridine. Les dérivés phénoliques (3) peuvent être obtenus par une réaction de type Friedel et Crafts à partir d'un phénol (2) et d'un dérivé dihalogéné (1) en présence d'un acide de Lewis par exemple le chlorure d'aluminium.

Les composés de formule I (b) peuvent être obtenus (figure 1) à partir du sel de sodium du dérivé thiol (6) par couplage avec un dérivé halogéné (7), de préférence un dérivé bromé ou iodé, en présence d'un catalyseur tel que certains complexes de métaux de transition dans un solvant alcoolique tel que l'alcool éthylique ou butylique.
Comme catalyseur on peut en particulier mentionner ceux dérivés du nickel ou du palladium par exemple les complexes de Ni ^{ll} avec diverses phosphines et le tétrakis(triphénylphosphine)palladium(0).

Les dérivés thiols (6) peuvent être obtenus à partir des dérivés phénoliques (3) via les dérivés dialkylthiocarbamates (4) et (5) selon les conditions générales décrites par M. Newman et H. Karnes dans J. Org. Chem 1966 31 3980-4.

Les dérivés de formules I (c) et I (d) peuvent être ensuite obtenus par oxydation du dérivé I (b), par exemple en employant l'acide méta chloroperbenzoïque.

Les composés de formule I (g) peuvent être obtenus (figure 2) à partir du dérivé acétylénique (13) par réaction avec le n-butyl lithium puis carboxylation en présence de CO₂. Les composés acétyléniques (13) peuvent être obtenus soit :
- à partir des dérivés aldéhydiques (11) (lorsque R₃ est un atome d'hydrogène), par réaction avec le tétrabromure de carbone et la triphenylphosphine pour donner un dérivé 2',2'-dibromostyrene qui est transformé en dérivé acétylènique par une base non nucléophile tel le n-butyllihium dans un solvant aprotic tel le tetrahydrofuranne.
- à partir des dérivés cétoniques (11) (lorsque R3 est un alkyl inférieur) par une suite de réactions comprenant le traitement avec une base tel le diisopropylamidure de lithium puis avec un chlorure de dialkylphosphate et de nouveau avec le diisopropylamidure de lithium.

Les composés de formule I (e) peuvent être obtenus (figure 2) à partir des dérivés aldéhydiques ou cétoniques (11), selon une réaction de type Horner avec un lithio dérivé d'un phosphonate (12). Puis par saponification dans la soude ou la potasse alcoolique, on obtient les composés de formule I (f).

Lorsque, dans la formule générale (I), X représente -NR₁₂ les composés I (h) peuvent être préparés suivant une réaction de type Ullman par déplacement nucléophilique direct d'un dérivé halogéné (7), de préférence iodé, par un dérivé aniline (8) en présence d'une base, telle que le carbonate de potassium ou la N-méthylmorpholine, et de cuivre.

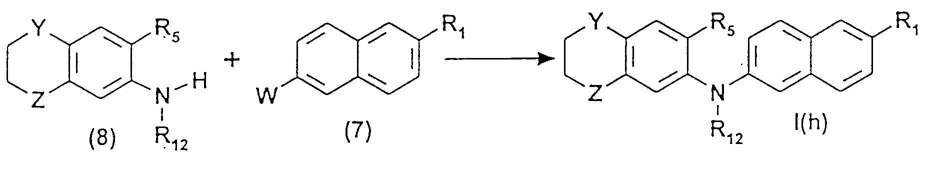

Dans les formules et réactions ci-dessus, ainsi que dans les figures, W représente un atome d'halogène.

Dans les formules et réactions ci-dessus, ainsi que dans les figures, R₁, R₂, R₃, R₄, R₅ , X, Y et Z ont les mêmes significations que celles données ci-avant pour la formule générale (I) ou en sont des dérivés convenablement protégés pour être compatibles avec les conditions de réaction. En particulier, lorsque R₁, R₂, R₄ et R₅ représentent le radical hydroxy, celui-ci est protégé de préférence sous forme de tert-butyldiméthylsilyloxy ou de méthoxyéthoxyméthoxy. La déprotection est effectuée soit en présence de fluorure de tétrabutylammonium, d'iodure de triméthylsilane ou en milieu acide (par exemple acide chlorhydrique).

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités des composés respectivement dans les domaines de la différenciation et de la prolifération cellulaire.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la super oxyde dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.
Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (4,5-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraenoïque et eicosa-5,8,11-trienoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration, plusieurs exemples d'obtention de composés actifs de formule (I) selon l'invention, ainsi que diverses formulations concrètes à base de tels composés.

### EXEMPLE 1

### Acide 6-(5,6,7,8-tétrahydro-5,5,8,8-tétraméthyl-2-naphtyloxy)naphtoïque

### (a) Méthyl 6-(5,6,7,8-tétrahydro-5,5,8,8-tétraméthyl-2-naphtyloxy)naphtoate

Dans un tricol et sous courant d'azote, on introduit 5,3 g (15 mmoles) de 5,6,7,8-tétrahydro-5,5,8,8-tétraméthyl-2-naphthol et 70 ml de pyridine, puis on ajoute par petites quantités 430 mg (15 mmoles) d'hydrure de sodium (80% dans l'huile). On agite pendant trente minutes, on ajoute sucessivement 4 g (15 mmoles) de méthyl 6-bromo-2-naphthoate et 4,6 g (22,5 mmoles) d'un complexe de bromure de cuivre et de diméthyl sulfure et chauffe à reflux pendant seize heures. On évapore à sec le milieu réactionnel, on reprend par l'eau et l'éther éthylique, décante la phase organique, sèche sur sulfate de magnésium, puis on évapore. Le résidu obtenu est purifié par chromatographie sur colonne de silice élué avec du dichlorométhane. Après évaporation des solvants, on recueille 3,1 g (53%) de l'ester méthylique attendu de point de fusion 141-4°C.

### (b) Acide 6-(5,6,7,8-tétrahydro-5,5,8,8-tétraméthyl-2-naphtyloxy)naphtoïque

Dans un ballon, on introduit 3,1 g (8 mmoles) de l'ester précédent, 40 ml de THF et 40 ml d'une solution de soude méthanolique (2N) et agite à température ambiante pendant huit heures. On évapore à sec le milieu réactionnel, reprend par l'eau, acidifie à pH 1, extrait avec de l'éther éthylique, décante la phase organique, sèche sur sulfate de magnésium, évapore. Le résidu obtenu est purifié par chromatographie sur colonne de silice élué avec un mélange de dichloromethane et d'éther éthylique (97-3). On recueille après évaporation des solvants, 1,3 g (44%) de l'acide attendu de point de fusion 231-2°C.

### EXEMPLE 2

### Acide 4-(5,6,7,8-tétrahydro-5,5,8,8-tétraméthyl-2-naphtyloxy)cinnamique

### (a) 4-(5,6,7,8-tétrahydro-5,5,8,8-tétraméthyl-2-naphtyloxy)benzaldéhyde

De manière analogue à l'exemple 1(a) par réaction de 5,3 g (15 mmoles) de 5,6,7,8-tétrahydro-5,5,8,8-tétraméthyl-2-naphthol avec 3,1 g (16,5 mmoles) de 4-bromobenzaldéhyde, on obtient, après purification par chromatographie sur colonne de silice élué avec un mélange de dichlorométhane et d'hexane (50-50), 2,4 g de l'aldéhyde attendu de point de fusion 75-6°C.

### (b) Ethyl 4-(5,6,7,8-tétrahydro-5,5,8,8-tétraméthyl-2-naphtyloxy)cinnamate

Dans un tricol et sous courant d'azote, on introduit 224 mg (8 mmoles) d'hydrure de sodium (80% dans l'huile) et 50 ml de diméthoxyethane et ajoute goutte à goutte une solution de 1,6 ml (8 mmoles) de triéthylphosphonoacétate dans 10 ml de diméthoxyéthane. On agite à température ambiante pendant une heure et à 0°C ajoute goutte à goutte une solution de 2,4 g (7,8 mmoles) de l'aldéhyde précèdent dans 20 ml de diméthoxyéthane. On agite à température ambiante pendant quatre heures, verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (30-70), on recueille 2,2 g (74%) de l'ester éthylique attendu.

### (c) acide 4-(5,6,7,8-tétrahydro-5,5,8,8-tétraméthyl-2-naphtyloxy)cinnamique

De manière analogue à l'exemple 1(b) à partir de 2,2 g (5,8 mmoles) d'éthyl 4-(5,6,7,8-tétrahydro-5,5,8,8-tétraméthyl-2-naphtyloxy)cinnamate, on obtient 1,5 g (75%) de l'acide attendu de point de fusion 220-1°C.

### EXEMPLE 3

### Acide β-méthyl-4-(5,6,7,8-tétrahydro-5,5,8,8-tétraméthyl-2-naphtyloxy)cinnamique

### (a) 4-(5,6,7,8-tétrahydro-5,5,8,8-tétraméthyl-2-naphtyloxy)acetophenone

De manière analogue à l'exemple 1(a) par réaction de 6,6 g (32 mmoles) de 5,6,7,8-tétrahydro-5,5,8,8-tétraméthyl-2-naphthol avec 5,4 g (27 mmoles) de 4-bromoacetophenone, on obtient 6,3 g (72%) de l'acetophenone attendu sous forme d'une huile légèrement jaune.

### (b) éthyl β-méthyl-4-(5,6,7,8-tétrahydro-5,5,8,8-tétraméthyl-2-naphtyloxy)cinnamate

De manière analogue à l'exemple 2(b) par réaction de 3 g (9,3 mmoles) de 4-(5,6,7,8-tétrahydro-5,5,8,8-tétraméthyl-2-naphtyloxy)acetophenone avec 2,5 g (11,2 mmoles) de triéthylphosphonoacétate, on obtient après chromatographie sur colonne de silice élué avec un mélange de dichlorométhane et d'hexane (30-70) 2,6 g (72%) d'ester éthylique sous forme d'une huile incolore.

### (c) acide β-méthyl-4-(5,6,7,8-tétrahydro-5,5,8,8-tétraméthyl-2-naphtyloxy)cinnamique

De manière analogue à l'exemple 1(b) à partir de 2,6 g (6,6 mmoles) de l'ester éthylique précédent, on obtient 1,1 g (46%) de l'acide attendu de point de fusion 156-7°C.

### EXEMPLE 4

### Acide 4-(5,6,7,8-tétrahydro-5,5,8,8-tétraméthyl-2-naphtyloxy)phenylpropiolique

### (a) 4-(5,6,7,8-tétrahydro-5,5,8,8-tétraméthyl-2-naphtyloxy)phenylacétylène

Dans un tricol et sous courant d'azote, on introduit 0,87 ml (6,2 mmoles) de diisopropylamine et 20 ml de THF. A -78°C, on ajoute goutte à goutte 2,5 ml (6,2 mmoles) de n-butyllithium (2,5 M dans l'hexane) et agite à cette même température pendant 15 minutes. On ajoute ensuite une solution de 2 g (6,2 mmoles) de 4-(5,6,7,8-tétrahydro-5,5,8,8-tétraméthyl-2-naphtyloxy)acétophénone préparé à l'exemple 3(a) dans 10 ml de THF. Après agitation pendant une heure à -78°C, la solution est traitée avec 0,9 ml (6,2 mmoles) de diethylchlorophosphate et on laisse remonter à température ambiante. Cette solution est transférée à une solution de diisopropylamidure de lithium (préparé en utilisant 1,74 ml (12,4 mmoles) de diisopropylamine et 5 ml (12,4 mmoles) de n-butyllithium (2,5 M dans l'hexane) dans 30 ml de THF à - 78°C. On laisse remonter à température ambiante et agite douze heures. On verse le milieu réactionnel dans eau glacée et ajuste le pH à 1 avec de l'acide chlorhydrique (5N). On extrait avec de l'éther éthylique, décante la phase organique, sèche sur sulfate de magnésium, évapore. Le résidu obtenu est purifié par chromatographie sur colonne de silice élué avec l'hexane. Après évaporation des solvants, on recueille 0,83 g (44%) du dérivé acetylenique sous forme d'une huile incolore.

### (b) acide 4-(5,6,7,8-tétrahydro-5,5,8,8-tétraméthyl-2-naphtyloxy)phenylpropiolique

Dans un tricol et sous courant d'azote, on introduit 0,82 g (2,7 mmoles) du dérivé acétylénique précédent et 10 ml de THF. A -78°C on ajoute goutte à goutte 1,85 ml (2,9 mmoles) de n-butyllithium (1,6 M dans l'hexane) et agite pendant trente minutes. A -78°C, on fait passer un courant de CO₂ pendant quinze minutes et laisse remonter à température ambiante. On verse le milieu réactionnel dans une solution aqueuse de chlorure d'ammonium, extrait avec de l'éther éthylique, décante la phase organique, sèche sur sulfate de magnésium, évapore.Le résidu obtenu est purifié par chromatographie sur colonne de silice élué avec du dichlorométhane. Après évaporation des solvants, on recueille 490 mg (53%) d'acide attendu de point de fusion 166-8°C.

### EXEMPLE 5

### Acide 6-(3,5,5,8,8-pentaméthyl-5,6,7,8-tétrahydro-2-naphtyloxy)naphtoïque

### (a) 3,5,5,8,8-pentaméthyl-5,6,7,8-tétrahydro-2-naphtol

Dans un tricol on introduit 50,8g (0,27 mole) de 2,5-dichloro-2,5-diméthylhexane 30 g (0,27 mole) de 2-méthylphénol et 500 ml de dichlorométhane. A 0°C on ajoute par petites quantités 14,8g (0,11 mole) de chlorure d'aluminium et agite à température ambiante pendant douze heures. On verse le milieu réactionnel dans l'eau glacée, extrait avec le dichlorométhane, décante la phase organique, lave à l'eau bicarbonatée, sèche sur sulfate de magnésium, évapore. Le résidu obtenu est trituré dans l'hexane, filtré, on recueille après sèchage 54,4 g (90%) de phénol attendu de point de fusion 125-6°C.

### (b) méthyl 6-(3,5,5,8,8-pentaméthyl-5,6,7,8-tétrahydro-2-naphtyloxy)naphtoate

De manière analogue à l'exemple 1(a) par réaction de 1,1 g (5 mmoles) de 3,5,5,8,8-pentaméthyl-5,6,7,8-tétrahydro-2-naphthol avec 1,1 g (4,1 mmoles) de méthyl 6-bromonaphtoate, on obtient 1,1 g (70%) d'ester méthylique attendu.

### (c) acide 6-(3,5,5,8,8-pentaméthyl-5,6,7,8-tétrahydro-2-naphtyloxy)naphtoïque

De manière analogue à l'exemple 1(b) à partir de 1,1 g (2,9 mmoles) de l'ester méthylique précédent, on obtient 600 mg (53%) d'acide attendu de point de fusion 237-9°C.

### EXEMPLE 6

### Acide 6-(5,6,7,8-tétrahydro-5,5,8,8-tétraméthyl-2-naphtylthio)naphtoïque

### (a) butyl 6-(5,6,7,8-tétrahydro-5,5,8,8-tétraméthyl-2-naphtylthio)naphtoate

Dans un ballon, on introduit 30 ml d'alcool N-butylique, on ajoute par petites quantités 500 mg (23 mmoles) de sodium et agite pendant trente minutes. On ajoute ensuite sucessivement 2 g (9,1 mmoles) de 5,6,7,8-tétrahydro-5,5,8,8-tétraméthyl-2-naphtylthiol, 2,4 g (9,1 mmoles) de méthyl 6-bromo-2-naphthoate et 420 mg (0,36 mmole) de tétrakis(triphénylphosphine)palladium(0), puis on chauffe à reflux pendant quatre heures. On évapore le milieu réactionnel, reprend par l'eau et l'acétate d'éthyle, décante la phase organique, lave à l'eau, sèche sur sulfate de magnésium, et évapore. Le résidu obtenu est purifié par chromatographie sur colonne de silice élué avec du dichlorométhane. Après évaporation des solvants, on recueille 2,2 g (55%) d'ester butylique.

### (b) acide 6-(5,6,7,8-tétrahydro-5,5,8,8-tétraméthyl-2-naphtylthio)naphtoïque

De manière analogue à l'exemple 1(b) à partir de 2,1 g (4,8 mmoles) de l'ester butylique précédent, on obtient 1,7 g (90%) de l'acide attendu de point de fusion 194-5°C.

### EXEMPLE 7

### Acide 6-(5,6,7,8-tétrahydro-5,5,8,8-tétraméthyl-2-naphtylsulfinyl)naphtoïque

### (a) butyl 6-(5,6,7,8-tétrahydro-5,5,8,8-tétraméthyl-2-naphtylsulfinyl)naphtoate

Dans un ballon on introduit 1,6 g (3,5 mmoles) de butyl 6-(5,6,7,8-tétrahydro-5,5,8,8-tétraméthyl-2-naphtylthio)naphtoate 30 ml de dichlorométhane et ajoute 1,1 g (3,5 mmoles) d'acide méta-chloroperbenzoïque. On agite à température ambiante pendant deux heures, verse le milieu réactionnel dans l'eau, extrait avec du dichlorométhane, décante la phase organique, sèche sur sulfate de magnésium, évapore. Le résidu est purifié par chromatographie sur colonne de silice élué avec du dichlorométhane. Après évaporation des solvants, on recueille 1,3 g (78%) de l'ester attendu.

### (b) acide 6-(5,6,7,8-tétrahydro-5,5,8,8-tétraméthyl-2-naphtylsulfinyl)naphtoïque

De manière analogue à l'exemple 1(b) à partir de 1,2 g (2,6 mmoles) de l'ester butylique précédent, on obtient 850 mg (81%) de l'acide attendu de point de fusion 183-5°C.

### EXEMPLE 8

### Acide 6-(5,6,7,8-tétrahydro-5,5,8,8-tétraméthyl-2-naphtylsulfonyl)naphtoïque

### (a) butyl 6-(5,6,7,8-tétrahydro-5,5,8,8-tétraméthyl-2-naphtylsulfonyl)naphtoate

Dans un ballon on introduit 2,2 g (5,1 mmoles) de butyl 6-(5,6,7,8-tétrahydro-5,5,8,8-tétraméthyl-2-naphthylthio)-naphthoate 30 ml de dichlorométhane et ajoute 4,3 g (12,6 mmoles) d'acide méta-chloroperbenzoïque. On agite à température ambiante pendant deux heures, verse le milieu réactionnel dans l'eau, extrait avec du dichlorométhane, décante la phase organique, sèche sur sulfate de magnésium, évapore. Le résidu est purifié par chromatographie sur colonne de silice élué avec du dichlorométhane. Après évaporation des solvants, on recueille 1,74 g (72%) de l'ester attendu.

### (b) acide 6-(5,6,7,8-tétrahydro-5,5,8,8-tétraméthyl-2-naphtylsulfonyl)naphtoïque

De manière analogue à l'exemple 1(b) à partir de 1,74 g (3,6 mmoles) de l'ester butylique précédent, on obtient 1,54 g (99%) de l'acide attendu de point de fusion 256-8°C..

### EXEMPLE 9

### Acide 6-(5,6,7,8-tétrahydro-5,5,8,8-tétraméthyl-2-naphtylamino)naphtoïque

### (a) méthyl 6-(5,6,7,8-tétrahydro-5,5,8,8-tétraméthyl-2-naphtylamino)naphtoate

Dans un ballon on introduit sucessivement 4,04 g (19,9 mmoles) de 5,6,7,8-tétrahydro-5,5,8,8-tétraméthyl-2-naphtylamine 5 g (19,9 mmoles) d'acide 6-bromo-2-naphtoïque 3,3 ml (30 mmoles) de N-méthylmorpholine 2,9 g (19,9 mmoles) de Cu₂O et 60 ml de dioxanne. On chauffe à reflux pendant 24 heures, verse le milieu réactionnel dans 60 ml d'acide chlorhydrique 5N, filtre le précipité, le lave à l'eau,sèche.Le solide est purifié par chromatographie sur colonne de silice élué avec l'acétate d'éthyle et après évaporation des solvants on recueille 1,2 g de l'acide attendu qui est transformé en ester méthylique par réaction dans 50 ml de'alcool méthylique en présence de 100 µl d'acide sulfurique concentré. On obtient 720 mg (10%) de méthyl 6-(5,6,7,8-tétrahydro-5,5,8,8-tétraméthyl-2-naphtylamino)naphthoate sous forme d'une huile marron.

### (b) acide 6-(5,6,7,8-tétrahydro-5,5,8,8-tétraméthyl-2-naphtylamino)naphtoïque

De manière analogue à l'exemple 1(b) à partir de 700 mg (1,8 mmole) de l'ester méthylique précédent, on obtient 670 mg (99%) de l'acide attendu de point de fusion 246-8°C.

### EXEMPLE 10

### Acide α-acétamido-4-(5,6,7,8-tétrahydro-5,5,8,8-tétraméthyl-2-naphtyloxy)cinnamique

### (a) 2-méthyl-4-[4-(5,6,7,8-tétrahydro-5,5,8,8-tétraméthyl-2-naphtyloxy)benzylidene]oxazol-5-one

Dans un ballon, on introduit 8,3 g (27 mmoles) de 4-(5,6,7,8-tétrahydro-5,5,8,8-tétraméthyl-2-naphtyloxy)benzaldéhyde 3,8 g (32 mmoles) de N-acétylglycine 2,9 g (35 mmoles) d'acétate de sodium et 13,8 g (135 mmoles) d'anhydride acétique. On chauffe à 120°C pendant huit heures, verse le milieu réactionnel dans l'eau, filtre le précipité et le lave avec une solution d'alcool éthylique et d'eau (50-50). Aprés purification par chromatographie sur colonne de silice élué avec un mélange de dichlorométhane et d'hexane (60-40), on recueille 8,9 g (85%) du produit attendu de point de fusion 145-7°C.

### (b) acide α-acetamido-4-(5,6,7,8-tétrahydro-5,5,8,8-tétraméthyl-2-naphtyloxy)cinnamique

Dans un ballon, on introduit 3,6 g (9 mmoles) du produit précédent 38 mg (0,4 mmoles) d'acétate de sodium et 100 ml d'un mélange d'eau et d'acétone (80-20). On chauffe à reflux pendant seize heures, refroidit le milieu réactionnel à température ambiante, filtre le solide, le lave à l'eau, sèche à 60°C. On recueille 3,5 g (92%) d'acide attendu de point de fusion 194-6°C.

### EXEMPLE 11

### Acide α-hydroxy-4-(5,6,7,8-tétrahydro-5,5,8,8-tétraméthyl-2-naphtyloxy)cinnamique

Dans un ballon, on introduit 1,6 g (4 mmoles) d'acide α-acetamido-4-(5,6,7,8-tétrahydro-5,5,8,8-tétraméthyl-2-naphtyloxy)cinnamique 25 ml d'acide chlorhydrique concentré et 25 ml d'alcool éthylique et chauffe à reflux pendant huit heures. On verse le milieu réactionnel dans l'eau, extrait avec de l'acétate d'éthyle, décante la phase organique, lave à l'eau, sèche sur sulfate de magnésium, évapore.Le résidu obtenu est trituré dans un mélange d'hexane et d'éther éthylique (80-20), filtré. On recueille 1,06 g (72%) de l'acide attendu de point de fusion 207-10°C.

### EXEMPLE 12

### Acide 6-(3,5,5,8,8-pentaméthyl-5,6,7,8-tétrahydro-2-naphtylthio)naphtoïque

### (a) O-3,5,5,8,8-pentaméthyl-5,6,7,8-tétrahydro-2-naphthyldimethylthiocarbamate

Dans un ballon et sous courant d'azote, on introduit 4,1 g (0,138 mole) d'hydrure de sodium (80% dans l'huile) et 200 ml de DMF. On refroidit à 0°C et ajoute goutte à goutte une solution de 25,2 g (0,115 mole) de 3,5,5,8,8-pentaméthyl-5,6,7,8-tétrahydro-2-naphthol dans 100 ml de DMF et agite jusqu'à cessation du dégagement gazeux, on ajoute ensuite une solution de 18,55 g (0,15 mole) de chlorure de dimethylthiocarbamoyle dans 200 ml de DMF et agite pendant huit heures à température ambiante. On verse le milieu réactionnel dans l'eau, extrait avec de l'acétate d'éthyle, décante la phase organique, lave à l'eau, sèche sur sulfate de magnésium, évapore. Le solide obtenu est purifié par chromatographie sur colonne de silice élué avec un mélange d'acétate d'éthyle et d'hexane (30-70). Après évaporation des solvants, on recueille 20 g (68%) du produit attendu de point de fusion 110-1°C.

### (b) S-3,5,5,8,8-pentaméthyl-5,6,7,8-tétrahydro-2-naphthyldimethylthiocarbamate

Dans un ballon et sous courant d'azote, on introduit 20,1 g (65,8 mmoles) du produit précédent et chauffe à 240°C pendant six heures. On extrait le milieu réactionnel avec du dichloromethane, lave à l'eau, décante la phase organique, sèche sur sulfate de magnésium, évapore. On recueille 18,1 g (90%) du produit attendu de point de fusion 138-9°C.

### (c) 3,5,5,8,8-pentaméthyl-5,6,7,8-tétrahydro-2-naphthylthiol

Dans un ballon, on introduit 23 g (75 mmoles) du produit précédent et 300 ml d'alcool methylique. On ajoute 30 g (75 mmoles) d'hydroxyde de sodium et chauffe à reflux pendant trois heures. On évapore le milieu réactionnel, reprend par l'eau, acidifie avec de l'acide chlorhydrique concentré, filtre. Le solide obtenu est lavé à l'eau, sèché, on recueille 18 g (99%) de 3,5,5,8,8-pentaméthyl-5,6,7,8-tétrahydro-2-naphthylthiol de point de fusion 97-8°C.

### (d) butyl 6-(3,5,5,8,8-pentaméthyl-5,6,7,8-tétrahydro-2-naphthylthio)naphtoate

De manière analogue à l'exemple 6 (a) par réaction de 4 g (17 mmoles) de 3,5,5,8,8-pentaméthyl-5,6,7,8-tétrahydro-2-naphthylthiol avec 4,5 g (17 mmoles) de méthyl 6-bromo-2-naphtoate, on obtient après chromatographie sur colonne de silice élué avec du dichlorométhane 5g (64%) d'ester butylique.

### (e) Acide 6-(3,5,5,8,8-pentaméthyl-5,6,7,8-tétrahydro-2-naphtylthio)naphtoïque

De manière analogue à l'exemple 1 (b) à partir de 2 g (4,3 mmoles) de l'ester butylique précédent, on obtient 1,22 g (70%) de l'acide attendu de point de fusion 257-8°C.

### EXEMPLE 13

### Acide 6-(3,5,5,8,8-pentaméthyl-5,6,7,8-tétrahydro-2-naphtylsulfonyl)naphtoïque

### (a) butyl 6-(3,5,5,8,8-pentaméthyl-5,6,7,8-tétrahydro-2-naphtylsulfonyl)naphtoate

De manière analogue à l'exemple 8(a) à partir de 2,7 g (5,8 mmoles) de butyl 4-(3,5,5,8,8-pentaméthyl-5,6,7,8-tétrahydro-2-naphthylthio)naphtoate, on obtient après purification par chromatographie sur colonne de silice élué avec le dichlorométhane 2,3 g (80%) d'ester butylique attendu.

### (b) Acide 6-(3,5,5,8,8-pentaméthyl-5,6,7,8-tétrahydro-2-naphtylsulfonyl)naphtoïque

De manière analogue à l'exemple 1(b) à partir de 2,3 g (4,6 mmoles) de l'ester butylique précédent, on obtient 1,32 g (65%) de l'acide attendu de point de fusion 274-6°C.

### EXEMPLE 14

### Acide 6-(3-bromo-5,6,7,8-tétrahydro-5,5,8,8-tétraméthyl-2-naphtyloxy)naphtoïque

### (a) 4-bromo-5,6,7,8-tétrahydro-5,5,8,8-tétraméthyl-2-naphtol

De manière analogue à l'exemple 5(a) par réaction de 3 g (16,4 mmoles) de 2,5-dichloro-2,5-diméthylhexane et 15 ml (16,4 mmoles) de 2-bromophenol et après purification par chromatographie sur colonne de silice élué avec un mélange d'acétate d'éthyle et d'heptane (10-90), on recueille 2,7 g (58%) de phénol attendu.

### (b) méthyl 6-(3-bromo-5,6,7,8-tétrahydro-5,5,8,8-tétraméthyl-2-naphtyloxy)naphtoate

De manière analogue à l'exemple 1(a) par réaction de 1 g (3,5 mmoles) de 3-bromo-5,6,7,8-tétrahydro-5,5,8,8-tétraméthyl-2-naphthol avec 0,78 g (2,9 mmoles) de méthyl 6-bromo-2-naphtoate, on obtient 460 mg (33%) d'ester méthylique attendu de point de fusion 145-6°C..

### (c) acide 6-(3-bromo-5,6,7,8-tétrahydro-5,5,8,8-tétraméthyl-2-naphtyloxy)naphtoïque

De manière analogue à l'exemple 1(b) à partir de 700 mg (1,7 mmoles) de l'ester méthylique précédent, on obtient 650 mg (95%) d'acide attendu de point de fusion 229-30°C.

### EXEMPLE 15

### 6-(5,6,7,8-tétrahydro-5,5,8,8-tétraméthyl-2-naphtyloxy)naphtalène méthanol

Dans un tricol, on introduit 2 g (5,1 mmoles) de méthyl 6-(5,6,7,8-tétrahydro-5,5,8,8-tétraméthyl-2-naphtyloxy)naphtoate (préparé à l'exemple 1(a)) et 60 ml de THF. Sous courant d'azote on introduit 300 mg (7,7 mmoles) d'hydrure double de lithium et d'aluminium et agite à température ambiante pendant deux heures. On ajoute du sulfate de sodium hydraté et agite jusqu'à hydrolyse de l'excès d'hydrure. On filtre le sel et èvapore le filtrat. On recueille 1,91 g (99%) de l'alcool attendu de point de fusion 131-3°C.

### EXEMPLE 16

### N-éthyl-6-(5,6,7,8-tétrahydro-5,5,8,8-tétraméthyl-2-naphtyloxy)naphtalène carboxamide

### (a) Chlorure de 6-(5,6,7,8-tétrahydro-5,5,8,8-tétraméthyl-2-naphtyloxy)naphtoyle

Dans un ballon, on introduit 1 g (2,6 mmoles) d'acide 6-(5,6,7,8-tétrahydro-5,5,8,8-tétraméthyl-2-naphtyloxy)naphtoïque (préparé à l'exemple 1(b)) 10 ml de toluène et 50 µl de DMF. On ajoute 230 µl (3,2 mmoles) de chlorure de thionyle et chauffe à 80°C pendant une heure. On évapore à sec et recueille le chlorure d'acide brut qui sera utilisé tel quel pour la suite de la synthèse.

### (b) N-éthyl-6-(5,6,7,8-tétrahydro-5,5,8,8-tétraméthyl-2-naphtyloxy)naphtalène carboxamide.

Dans un ballon, on introduit 520 µl (6,4 mmoles) d'éthylamine (70% dans l'eau) et 5 ml de THF. On ajoute goutte à goutte, une solution de 1,05 g (2,6 mmoles) du chlorure d'acide précédent dans 5 ml de THF et agite à température ambiante une heure. On verse le milieu réactionnel dans l'eau, extrait avec de l'acétate d'éthyle, décante la phase organique, sèche sur sulfate de magnésium, évapore. On recueille 1 g (93%) de l'amide attendu de point de fusion 168-70°C.

### EXEMPLE 17

### 6-(5,6,7,8-tétrahydro-5,5,8,8-tétraméthyl-2-naphtyloxy)naphtalène carboxamide

De manière analogue à l'exemple 16 (b) par réaction de 671 mg (1,7 mmole) de chlorure de 6-(5,6,7,8-tétrahydro-5,5,8,8-tétraméthyl-2-naphtyloxy)naphtoyle avec 10 ml d'ammoniaque (32%); on obtient 560 mg (88%) de l'amide attendu de point de fusion 175-6°C.

### EXEMPLE 18

### Morpholide de l'acide 6-(5,6,7,8-tétrahydro-5,5,8,8-tétraméthyl-2-naphtyloxy)naphtoïque

De manière analogue à l'exemple 16 (b) par réaction de 593 mg (1,5 mmole) de chlorure de 6-(5,6,7,8-tétrahydro-5,5,8,8-tétraméthyl-2-naphtyloxy)naphtoyle avec 320 µl (3,6 mmoles) de morpholine; on obtient 470 mg (70%) de l'amide attendu de point de fusion 118-20°C.

### EXEMPLE 19

### 6-(5,6,7,8-tétrahydro-5,5,8,8-tétraméthyl-2-naphtyloxy)naphtalène carboxaldéhyde

Dans un ballon, on introduit 100 ml de dichlorométhane et 1,7 g (4,5 mmoles) de pyridinium dichromate. On refroidit à 0°C et ajoute goutte à goutte une solution de 1,2 g (3,3 mmoles) de 6-(5,6,7,8-tétrahydro-5,5,8,8-tétraméthyl-2-naphtyloxy)naphtalène méthanol dans 20 ml de dichlorométhane. On agite à température ambiante une heure, filtre le milieu réactionnel sur silice, évapore le filtat à sec, triture le solide dans l'heptane, filtre. On recueille 1,07 g (90%) de l'aldéhyde attendu de point de fusion 123-4°C.

### EXEMPLE 20

Dans cet exemple, on a illustré diverses formulations concrètes à base des composés selon l'invention.

### A- VOIE ORALE

| (a) Comprimé de 0,2 g | |
|---|---|
| - Composé de l'exemple 1 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |

| (b) Suspension buvable en ampoules de 5 ml | |
|---|---|
| - Composé de l'exemple 2 | 0,001 g |
| - Glycérine | 0,500 g |
| - Sorbitol à 70% | 0,500 g |
| - Saccharinate de sodium | 0,010 g |
| - Parahydroxybenzoate de méthyle | 0,040 g |
| - Arome qs | |
| - Eau purifiée qsp | 5 ml |

| (c) Comprimé de 0,8 g | |
|---|---|
| - Composé de l'exemple 6 | 0,500 g |
| - Amidon prégélatinisé | 0,100 g |
| - Cellulose microcristalline | 0,115 g |
| - Lactose | 0,075 g |
| - Stéarate de magnésium | 0,010 g |

| (d) Suspension buvable en ampoules de 10 ml | |
|---|---|
| - Composé de l'exemple 4 | 0,05 g |
| - Glycérine | 1,000g |
| - Sorbitol à 70% | 1,000g |
| - Saccharinate de sodium | 0,010 g |
| - Parahydroxybenzoate de méthyle | 0,080 g |
| - Arome qs | |
| - Eau purifiée qsp | 10 ml |

### B- VOIE TOPIQUE

| (a) Onguent | |
|---|---|
| - Composé de l'exemple 1 | 0,020 g |
| - Myristate d'isopropyle | 81,700 g |
| - Huile de vaseline fluide | 9,100 g |
| - Silice ("Aérosil 200" vendue par DEGUSSA) | 9,180 g |

| (b) Onguent | |
|---|---|
| - Composé de l'exemple 6 | 0,300 g |
| - Vaseline blanche codex | 100 g |

| (c) Crème Eau-dans-Huile non ionique | |
|---|---|
| - Composé de l'exemple 1 | 0,100 g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |

| (d) Lotion | |
|---|---|
| - Composé de l'exemple 1 | 0,100 g |
| - Polyéthylène glycol (PEG 400) | 69,900 g |
| - Ethanol à 95% | 30,000 g |

| (e) Onguent hydrophobe | |
|---|---|
| - Composé de l'exemple 2 | 0,300 g |
| - Mirystate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) | 100g |

| (f) Crème Huile-dans-Eau non ionique | |
|---|---|
| - Composé de l'exemple 4 | 0,500 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile | 100 g |

## Revendications

1. Composés bicycliques aromatiques , caractérisés par le fait qu'ils répondent à la formule générale (I) suivante : 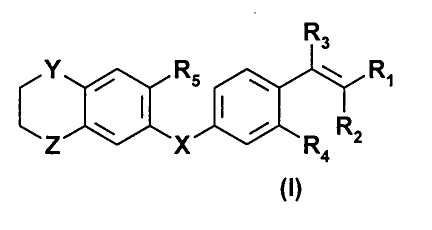 dans laquelle:
* R₁ représente
(i) un atome d'hydrogène
(ii) le radical -CH₃
(iii) le radical -CH₂OR₆
(iv) le radical -O-R₈,uniquement lorsque R₂ et R₄ forment avec le cycle benzénique adjacent un cycle naphthalénique;
(v) le radical -CO-R₁₀
(vi) un radical -S(O)p-R₁₂
R₆, R₈, R₁₀, R₁₂ et p ayant les significations données ci-après,
* R2 et R3 sont soit indépendants, soit pris ensemble,
- dans le cas où ils sont indépendants :
R₂ représente un atome d'hydrogène, un radical alkyle inférieur: (C₁-C₁₂), un radical hydroxy,un radical -OR₇, un radical -O-COR₇, un radical amino ou un radical -NH-COR₇;
R3 représente un atome d'hydrogène ou un radical alkyle inférieur: (C₁-C₁₂);
- dans le cas où ils sont pris ensemble :
R₂ et R3 forment avec la liaison éthylénique une liaison acétylénique;
* R₂ et R₄ sont soit indépendants, soit pris ensemble,
- dans le cas où ils sont indépendants :
R₂ a la signification donnée ci-dessus;
R₄ représente un atome d'hydrogène, un radical alkyle linéaire ou ramifié ayant de 1 à 20 atomes de carbone, un radical hydroxy, un radical - OR₇, un radical -O-COR₇;
- dans le cas où ils sont pris ensemble :
R₂ et R₄ forment avec le cycle benzénique adjacent un cycle naphthalénique.
R₇ ayant la signification donnée ci-après;
* R₅ représente un atome d'hydrogène, un atome d'halogène, un radical alkyle inférieur: (C₁-C₁₂), un radical NO₂, un radical hydroxy, un radical -OR₇, un radical -O-COR₇, ou bien encore un radical 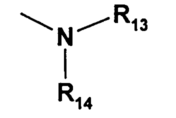 R₇, R₁₃ et R₁₄ ayant les significations données ci-après;
* X représente -O-, -S(O)ₜ- ou -NR₁₂-,
t et R₁₂ ayant les significations données ci-après;
* Y et Z, identiques ou différents, représentent -CR₁₃R₁₄-, -O-, -S(O)ₜ- , avec la condition que Y et Z ne sont pas simultanément un atome d'oxygène ou simultanément un radical -S(O)ₜ;
t, R₁₃ et R₁₄ ayant les significations données ci-après;
étant entendu que dans tout ce qui précède :
R₆ représente un atome d'hydrogène, un radical alkyle inférieur: (C₁-C₁₂), un radical -COR₇ ;
R₇ représente un radical alkyle inférieur: (C₁-C₁₂)
R₈ représente un atome d'hydrogène, un radical alkyle inférieur: (C₁-C₁₂), un radical -(CH2)m-(CO)n-R₉
R₉ représente un radical alkyle inférieur ou un hétérocycle
R₁₀ représente:
(a) un atome d'hydrogène;
(b) un radical alkyle inférieur: (C₁-C₁₂);
(c) un radical 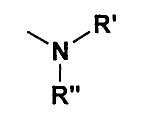
(d) un radical-O-R₁₁
R₁₁ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre ou un reste d'amino acide ou de peptide;
R₁₂ représente un atome d'hydrogène, un radical alkyle inférieur: (C₁-C₁₂)
R₁₃ et R₁₄ représentent un atome d'hydrogène, un radical alkyle inférieur: (C₁-C₁₂),
m est un entier variant de 1 à 3;
n est 0 ou 1;
p est 0 ou un entier variant de 1 à 3;
t est 0, 1 ou 2;
R' et R'' représentent indépendamment un atome d'hydrogène, un radical alkyle inférieur: (C₁-C₁₂), un radical mono ou polyhydroxyalkyle, un radical aryle éventuellement substitué ou un reste d'amino acide ou de sucre ou encore pris ensemble forment un hétérocycle;
ainsi que leurs sels et leurs isomères optiques et géométriques.

2. Composés selon la revendication 1, caractérisés par le fait qu'ils se présentents sous forme de sels d'un métal alcalin ou alcalino-terreux, ou encore de zinc ou d'une amine organique.

3. Composés selon l'une des revendications 1 ou 2, caractérisés par le fait que les radicaux alkyles inférieurs sont choisis parmi le groupe constitué par les radicaux méthyle, éthyle, isopropyle, butyle, tertiobutyle, hexyle, nonyle et dodécyle .

4. Composés selon l'une des revendications précédentes, caractérisés en ce que les radicaux alkyles linéaires ou ramifiés ayant de 1 à 20 atomes de carbone sont choisis parmi le groupe constitué par les radicaux méthyle, éthyle, propyle, 2-éthyl-hexyle, octyle, dodécyle, hexadécyle et octadécyle.

5. Composés selon l'une des revendications précédentes, caractérisés en ce que les radicaux monohydroxyalkyles sont choisis parmi le groupe constitué par les radicaux 2-hydroxyéthyle, 2-hydroxypropyle ou 3-hydroxypropyle.

6. Composés selon l'une des revendications précédentes, caractérisés en ce que les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une des revendications précédentes, caractérisés en ce que le radical aryle est un radical phényle éventuellement substitué par au moins un atome d'halogène, un hydroxyle ou une fonction nitro.

8. Composés selon l'une des revendications précédentes, caractérisés en ce que les radicaux aralkyles sont choisis parmi le groupe constitué par les radicaux benzyle ou phénéthyle éventuellement substitués par au moins un atome d'halogène, un hydroxyle ou une fonction nitro.

9. Composés selon l'une des revendications précédentes, caractérisés en ce que les radicaux alkényles sont choisis dans le groupe constitué par les radicaux contenant de 2 à 5 atomes de carbone et présentant une ou plusieurs insaturations éthyléniques, et en particulier le radical allyle.

10. Composés selon l'une des revendications précédentes, caractérisés en ce que les restes de sucre sont choisis dans le groupe constitué par les restes de glucose, de galactose ou de mannose, ou d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les atomes d'halogène sont choisis dans le groupe constitué par le fluor, le chlore et le brome.

15. Composés selon la revendication 1, caractérisés par le fait qu'ils sont pris, seuls ou en mélanges, dans le groupe constitué par :
Acide 6-(5,6,7,8-tétrahydro-5,5,8,8-tétraméthyl-2-naphtyloxy)naphtoïque
Acide 4-(5,6,7,8-tétrahydro-5,5,8,8-tétraméthyl-2-naphtyloxy)cinnamique
Acide β-methyl-4-(5,6,7,8-tétrahydro-5,5,8,8-tétraméthyl-2-naphtyloxy)cinnamique
Acide 4-(5,6,7,8-tétrahydro-5,5,8,8-tétraméthyl-2-naphtyloxy)phenylpropiolique
Acide 6-(3,5,5,8,8-pentaméthyl-5,6,7,8-tétrahydro-2-naphtyloxy)naphtoïque
Acide 6-(5,6,7,8-tétrahydro-5,5,8,8-tétraméthyl-2-naphtylthio)naphtoïque
Acide 6-(5,6,7,8-tétrahydro-5,5,8,8-tétraméthyl-2-naphtylsulfinyl)naphtoïque
Acide 6-(5,6,7,8-tétrahydro-5,5,8,8-tétraméthyl-2-naphtylsulfonyl)naphtoïque
Acide 6-(5,6,7,8-tétrahydro-5,5,8,8-tétraméthyl-2-naphtylamino)naphtoïque
Acide α-hydroxy-4-(5,6,7,8-tétrahydro-5,5,8,8-tétraméthyl-2-naphtyloxy)cinnamique
Acide α-acetamido-4-(5,6,7,8-tétrahydro-5,5,8,8-tétraméthyl-2-naphtyloxy)cinnamique
Acide 6-(3,5,5,8,8-pentaméthyl-5,6,7,8-tétrahydro-2-naphtylthio)naphtoïque
Acide 6-(3,5,5,8,8-pentaméthyl-5,6,7,8-tétrahydro-2-naphtylsulfonyl)naphtoïque
Acide 6-(3-bromo-5,6,7,8-tétrahydro-5,5,8,8-tétraméthyl-2-naphtyloxy)naphtoïque
6-(5,6,7,8-tétrahydro-5,5,8,8-tétraméthyl-2-naphtyloxy)naphtalène méthanol
N-éthyl-6-(5,6,7,8-tétrahydro-5,5,8,8-tétraméthyl-2-naphtyloxy)naphtalène carboxamide
6-(5,6,7,8-tétrahydro-5,5,8,8-tétraméthyl-2-naphtyloxy)naphtalène carboxamide
Morpholide de l'acide 6-(5,6,7,8-tétrahydro-5,5,8,8-tétraméthyl-2-naphtyloxy)naphtoïque
6-(5,6,7,8-tétrahydro-5,5,8,8-tétraméthyl-2-naphtyloxy)naphtalène carboxaldéhyde

16. Composés selon la revendication 1, caractérisés par le fait qu'ils présentent l'une au moins des caractéristiques suivantes :
- R₁ est un radical -CO-R₁₀
- X représente -O-, -S-, -NR₁₂-
- R₂ représente un atome d'hydrogène ou un radical alkyle inférieur
- R₂ et R₄ forment avec le cycle benzénique adjacent un cycle naphtalénique
- R₂ et R₃ forment avec la liaison éthylénique une liaison acétylénique

17. Composés selon l'une quelconque des revendications précédentes pour une utilisation comme médicament.

18. Composés selon la revendication 17 pour une utilisation comme médicament destiné au traitement des affections dermatologiques, rhumatismales, respiratoires, cardiovasculaires et ophtalmologiques.

19. Utilisation de l'un au moins des composés définis aux revendications 1 à 16 pour la fabrication d'un médicament destiné au traitement des affections dermatologiques, rhumatismales, respiratoires, cardiovasculaires et ophtalmologiques.

20. Composition pharmaceutique, caractérisée par le fait qu'elle comprend, dans un support pharmaceutiquement acceptable, au moins l'un des composés tels que définis à l'une quelconque des revendications 1 à 16.

21. Composition selon la revendication 20, caractérisée en ce que la concentration en composé(s) selon l'une des revendication 1 à 16 est comprise entre 0,001 % et 5 % en poids par rapport à l'ensemble de la composition.

22. Composition cosmétique, caractérisée par le fait qu'elle comprend, dans un support cosmétiquement acceptable, au moins l'un des composés tels que définis à l'une quelconque des revendications 1 à 16.

23. Composition selon la revendication 22, caractérisée en ce que la concentration en composé(s) selon l'une des revendications 1 à 16 est comprise entre 0,001 % et 3 % en poids par rapport à l'ensemble de la composition.

24. Utilisation d'une composition cosmétique telle que définie à l'une des revendications 22 ou 23 pour l'hygiène corporelle ou capillaire.

## Claims

1. Bicyclic aromatic compounds, characterized in that they correspond to the following general formula (I): 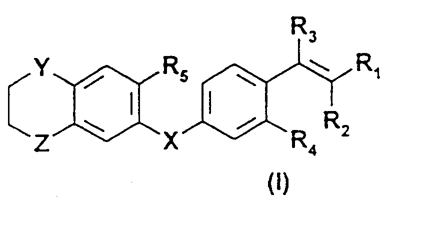 in which:
* R₁ represents
(i) a hydrogen atom
(ii) the -CH₃ radical
(iii) a radical -CH₂OR₆
(iv) a radical -O-R₈, only when R₂ and R₄ form, with the adjacent benzene ring, a naphthalene ring system;
(v) a radical -CO-R₁₀
(vi) a radical -S(O)ₚ-R₁₂,
R₆, R₈, R₁₀, R₁₂ and p having the meanings given below,
* R₂ and R₃ are, either independently of one another or taken together,
- in the case where they are independent of one another:
R₂ represents a hydrogen atom, a (C₁-C₁₂) lower alkyl radical, a hydroxyl radical, a radical -OR₇, a radical -O-COR₇, an amino radical or a radical -NH-COR₇;
R₃ represents a hydrogen atom or a (C₁-C₁₂) lower alkyl radical;
- in the case where they are taken together:
R₂ and R₃ form, with the ethylenic bond, an acetylenic bond;
* R₂ and R₄ are, either independently of one another or taken together,
- in the case where they are independent of one another:
R₂ has the meaning given above;
R₄ represents a hydrogen atom, a linear or branched alkyl radical having 1 to 20 carbon atoms, a hydroxyl radical, a radical -OR₇, a radical -O-COR₇;
- in the case where they are taken together:
R₂ and R₄ form, with the adjacent benzene ring, a naphthalene ring system,
R₇ having the meaning given below;
* R₅ represents a hydrogen atom, a halogen atom, a (C₁-C₁₂) lower alkyl radical, a NO₂ radical, a hydroxyl radical, a radical -OR₇, a radical -O-COR₇, or else a radical 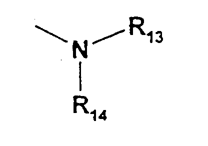 R₇, R₁₃ and R₁₄ having the meanings given below;
* X represents -O-, -S(O)ₜ- or -NR₁₂-,
t and R₁₂ having the meanings given below;
* Y and Z, which are identical or different, represent -CR₁₃R₁₄-, -O-, -S(O)ₜ-, with the proviso that Y and Z are not simultaneously an oxygen atom or simultaneously a radical -S(O)ₜ-;
t, R₁₃ and R₁₄ having the meanings given below;
it being understood that, in all of the above:
R₆ represents a hydrogen atom, a (C₁-C₁₂) lower alkyl radical, a radical -COR₇;
R₇ represents a (C₁-C₁₂) lower alkyl radical
R₈ represents a hydrogen atom, a (C₁-C₁₂) lower alkyl radical, a radical -(CH₂)ₘ-(CO)ₙ-R₉
R₉ represents a lower alkyl radical or a heterocycle
R₁₀ represents:
(a) a hydrogen atom;
(b) a (C₁-C₁₂) lower alkyl radical;
(c) a radical 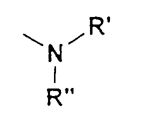
(d) a radical -O-R₁₁
R₁₁ represents a hydrogen atom, a linear or branched alkyl radical having 1 to 20 carbon atoms, an alkenyl radical, a mono- or polyhydroxyalkyl radical, an optionally substituted aryl or aralkyl radical or a sugar radical or an amino-acid residue or peptide residue;
R₁₂ represents a hydrogen atom, a lower alkyl radical;
R₁₃ and R₁₄ represent a hydrogen atom, a (C₁-C₁₂) lower alkyl radical;
m is an integer ranging from 1 to 3;
n is 0 or 1;
p is 0 or an integer ranging from 1 to 3;
t is 0, 1 or 2;
R' and R'' represent, independently of one another, a hydrogen atom, a (C₁-C₁₂) lower alkyl radical, a mono- or polyhydroxyalkyl radical, an optionally substituted aryl radical or an amino-acid residue or sugar radical or else, taken together, form a heterocycle;
and also their salts and their optical and geometric isomers.

2. Compounds according to Claim 1, characterized in that they are in the form of salts of an alkali metal or alkaline-earth metal, or else salts of zinc or of an organic amine.

3. Compounds according to either of Claims 1 and 2, characterized in that the lower alkyl radicals are chosen from the group consisting of the radicals methyl, ethyl, isopropyl, butyl, tert-butyl, hexyl, nonyl and dodecyl.

4. Compounds according to one of the preceding claims, characterized in that the linear or branched alkyl radicals having 1 to 20 carbon atoms are chosen from the group consisting of the radicals methyl, ethyl, propyl, 2-ethylhexyl, octyl, dodecyl, hexadecyl and octadecyl.

5. Compounds according to one of the preceding claims, characterized in that the monohydroxyalkyl radicals are chosen from the group consisting of the radicals 2-hydroxyethyl, 2-hydroxypropyl and 3-hydroxypropyl.

6. Compounds according to one of the preceding claims, characterized in that the polyhydroxyalkyl radicals are chosen from the group consisting of the radicals 2,3-dihydroxypropyl, 2,3,4-trihydroxybutyl, 2,3,4,5-tetrahydroxypentyl and the radical of pentaerythritol.

7. Compounds according to one of the preceding claims, characterized in that the aryl radical is a phenyl radical which is optionally substituted with at least one halogen atom, a hydroxyl or a nitro functional group.

8. Compounds according to one of the preceding claims, characterized in that the aralkyl radicals are chosen from the group consisting of the radicals benzyl and phenethyl which are optionally substituted with at least one halogen atom, a hydroxyl or a nitro functional group.

9. Compounds according to one of the preceding claims, characterized in that the alkenyl radicals are chosen from the group consisting of the radicals containing 2 to 5 carbon atoms and having one or more ethylenic unsaturations, and in particular the allyl radical.

10. Compounds according to one of the preceding claims, characterized in that the sugar radicals are chosen from the group consisting of the radicals of glucose, galactose or mannose, or of glucuronic acid.

11. Compounds according to any one of the preceding claims, characterized in that the amino-acid residues are chosen from the group consisting of the residues derived from lysine, glycine or aspartic acid.

12. Compounds according to any one of the preceding claims, characterized in that the peptide residues are chosen from the group consisting of the residues of dipeptides or tripeptides.

13. Compounds according to any one of the preceding claims, characterized in that the heterocyclic radicals are chosen from the group consisting of the radicals piperidino, morpholino, pyrrolidino and piperazino which are optionally substituted in position 4 with a C₁-C₆ alkyl or mono- or polyhydroxyalkyl radical.

14. Compounds according to any one of the preceding claims, characterized in that the halogen atoms are chosen from the group consisting of fluorine, chlorine and bromine.

15. Compounds according to Claim 1, characterized in that they are taken, individually or in mixtures, from the group consisting of:
6-(5,6,7,8-tetrahydro-5,5,8,8-tetramethyl-2-naphthyloxy)naphthoic acid
4-(5,6,7,8-tetrahydro-5,5,8,8-tetramethyl-2-naphthyloxy)cinnamic acid
β-methyl-4-(5,6,7,8-tetrahydro-5,5,8,8-tetramethyl-2-naphthyloxy)cinnamic acid
4-(5,6,7,8-tetrahydro-5,5,8,8-tetramethyl-2-naphthyloxy)phenylpropiolic acid
6-(3,5,5,8,8-pentamethyl-5,6,7,8-tetrahydro-2-naphthyloxy)naphthoic acid
6-(5,6,7,8-tetrahydro-5,5,8,8-tetramethyl-2-naphthylthio)naphthoic acid
6-(5,6,7,8-tetrahydro-5,5,8,8-tetramethyl-2-naphthylsulphinyl)naphthoic acid
6-(5,6,7,8-tetrahydro-5,5,8,8-tetramethyl-2-naphthylsulphonyl)naphthoic acid
6-(5,6,7,8-tetrahydro-5,5,8,8-tetramethyl-2-naphthylamino)naphthoic acid
α-hydroxy-4-(5,6,7,8-tetrahydro-5,5,8,8-tetramethyl-2-naphthyloxy)cinnamic acid
α-acetamido-4-(5,6,7,8-tetrahydro-5,5,8,8-tetramethyl-2-naphthyloxy)cinnamic acid
6-(3,5,5,8,8-pentamethyl-5,6,7,8-tetrahydro-2-naphthylthio)naphthoic acid
6-(3,5,5,8,8-pentamethyl-5,6,7,8-tetrahydro-2-naphthylsulphonyl)naphthoic acid
6-(3-bromo-5,6,7,8-tetrahydro-5,5,8,8-tetramethyl-2-naphthyloxy)naphthoic acid
6-(5,6,7,8-tetrahydro-5,5,8,8-tetramethyl-2-naphthyloxy)naphthalenemethanol
N-ethyl-6-(5,6,7,8-tetrahydro-5,5,8,8-tetramethyl-2-naphthyloxy)naphthalenecarboxamide
6-(5,6,7,8-tetrahydro-5,5,8,8-tetramethyl-2-naphthyloxy)naphthalenecarboxamide
6-(5,6,7,8-tetrahydro-5,5,8,8-tetramethyl-2-naphthyloxy)naphthoic acid morpholide and
6-(5,6,7,8-tetrahydro-5,5,8,8-tetramethyl-2-naphthyloxy)naphthalenecarbaldehyde.

16. Compounds according to Claim 1, characterized in that they have at least one of the following characteristics:
- R₁ is a radical -CO-R₁₀;
- X represents -O-, -S-, -NR₁₂-;
- R₂ represents a hydrogen atom or a lower alkyl radical;
- R₂ and R₄ form, with the adjacent benzene ring, a naphthalene ring system.
- R₂ and R₃ form, with the ethylenic bond, an acetylenic bond.

17. Compounds according to any one of the preceding claims, for use as medicinal products.

18. Compounds according to Claim 17 for use as medicinal products which are intended for the treatment of dermatological, rheumatic, respiratory, cardiovascular and ophthalmological conditions.

19. Use of at least one of the compounds defined in Claims 1 to 16 for the manufacture of a medicinal product which is intended for the treatment of dermatological, rheumatic, respiratory, cardiovascular and ophthalmological conditions.

20. Pharmaceutical composition, characterized in that it comprises, in a pharmaceutically acceptable carrier, at least one of the compounds as defined in any one of Claims 1 to 16.

21. Composition according to Claim 20, characterized in that the concentration of compound(s) according to one of Claims 1 to 16 is between 0.001 % and 5 % by weight, relative to the composition in its entirety.

22. Cosmetic composition, characterized in that it comprises, in a cosmetically acceptable carrier, at least one of the compounds as defined in any one of Claims 1 to 16.

23. Composition according to Claim 22, characterized in that the concentration of compound(s) according to one of Claims 1 to 16 is between 0.001 % and 3 % by weight, relative to the composition in its entirety.

24. Use of a cosmetic composition as defined in either of Claims 22 and 23 for body or hair hygiene.

## Patentansprüche

1. Bicyclische aromatische Verbindungen, dadurch gekennzeichnet, daß sie die nachstehende allgemeine Formel (I) aufweisen, 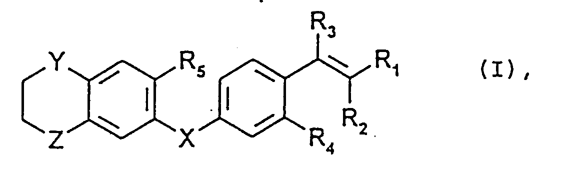 in der bedeuten:
• R₁
(i) ein Wasserstoffatom,
(ii) die Gruppe -CH₃,
(iii) die Gruppe -CH₂OR₆,
(iv) die Gruppe -O-R₈, jedoch lediglich, wenn R₂ und R₄ mit dem benachbarten Benzolring einen Naphthalinring bilden,
(v) die Gruppe -CO-R₁₀
oder
(vi) eine Gruppe -S(O)p-R₁₂,
wobei R₆, R₈, R₁₀, R₁₂ und p die unten angegebene Bedeutung besitzen,
• R₂ und R₃, die voneinander unabhängig oder zusammengehörig sind:
- wenn sie voneinander unabhängig sind:
R₂ ein Wasserstoffatom, eine niedere Alkylgruppe (C₁ bis C₁₂), eine Hydroxygruppe, eine Gruppe -OR₇, eine Gruppe -O-COR₇, eine Aminogruppe oder eine Gruppe -NH-COR₇ und
R₃ ein Wasserstoffatom oder eine niedere Alkylgruppe (C₁ bis C₁₂);
- wenn sie zusammengehörig sind:
R₂ und R₃ bilden mit der ethylenischen Doppelbindung eine Dreifachbindung;
• R₂ und R₄, die voneinander unabhängig oder zusammengehörig sind:
- wenn sie voneinander unabhängig sind:
R₂ dasselbe wie oben definiert und
R₄ ein Wasserstoffatom, eine geradkettige oder verzweigte Alkylgruppe mit 1 bis 20 C-Atomen, Hydroxygruppe, eine Gruppe -OR₇ oder eine Gruppe -O-COR₇;
- wenn sie zusammengehörig sind:
R₂ und R₄ bilden mit dem benachbarten Benzolring einen Naphthalinring,
wobei R₇ die unten angegebene Bedeutung besitzt;
• R₅ ein Wasserstoffatom, ein Halogenatom, eine niedere Alkylgruppe (C₁ bis C₁₂), eine Gruppe NO₂, eine Hydroxygruppe, eine Gruppe -OR₇, eine Gruppe -O-COR₇ oder eine Gruppe 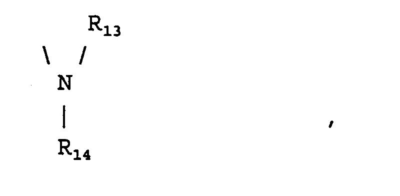 wobei R₇, R₁₃ und R₁₄ die unten angegebene Bedeutung besitzen;
• X -O-, -S(O)ₜ- oder -NR₁₂-,
wobei t und R₁₂ die unten angegebene Bedeutung besitzen,
und
• Y und Z, die gleich oder voneinander verschieden sind, -CR₁₃R₁₄-, -O- oder -S(O)ₜ-,
mit der Maßgabe, daß Y und Z nicht gleichzeitig ein Sauerstoffatom oder gleichzeitig eine Gruppe -S(O)ₜ- bedeuten,
wobei t, R₁₃ und R₁₄ die unten angegebene Bedeutung besitzen;
wobei die oben angeführten Abkürzungen folgende Bedeutung haben:
R₆ ein Wasserstoffatom, eine niedere Alkylgruppe (C₁ bis C₁₂) oder eine Gruppe -COR₇,
R₇ eine niedere Alkylgruppe (C₁ bis C₁₂),
R₈ ein Wasserstoffatom, eine niedere Alkylgruppe (C₁ bis C₁₂) oder eine Gruppe -(CH₂)m-(CO)n-R₉, wobei R₉ eine niedere Alkylgruppe oder einen Heterocyclus darstellt,
R₁₀
(a) ein Wasserstoffatom,
(b) eine niedere Alkylgruppe (C₁ bis C₁₂),
(c) eine Gruppe 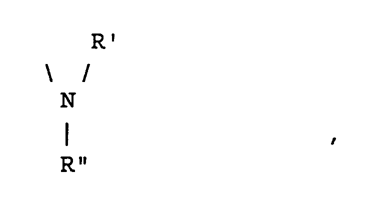 worin R^{'} und R'' unabhängig ein Wasserstoffatom, eine niedere Alkylgruppe (C₁ bis C₁₂), eine Mono- oder Polyhydroxyalkylgruppe, eine ggf. substituierte Arylgruppe oder einen Aminosäurerest oder einen Zuckerrest bedeuten oder zusammen einen Heterocyclus bilden,
oder
(d) eine Gruppe -O-R₁₁,
wobei R₁₁ ein Wasserstoffatom, eine geradkettige oder verzweigte Alkylgruppe mit 1 bis 20 C-Atomen, eine Alkenylgruppe, eine Mono- oder Polyhydroxyalkylgruppe oder eine Arylgruppe oder Aralkylgruppe, die ggf. mit einem Zuckerrest oder einem Aminosäurerest oder einem Peptidrest substituiert sind, bedeutet,
R₁₂ ein Wasserstoffatom oder eine niedere Alkylgruppe (C₁ bis C₁₂),
R₁₃ und R₁₄ ein Wasserstoffatom oder eine niedere Alkylgruppe (C₁ bis C₁₂),
m eine ganze Zahl von 1 bis 3,
n 0 oder 1,
p 0 oder eine ganze Zahl von 1 bis 3
und
t 0, 1 oder 2,
sowie ihre Salze und ihre optischen Isomeren und geometrischen Strukturisomeren.

2. Verbindungen nach Anspruch 1, dadurch gekennzeichnet, daß sie in Form von Alkalimetallsalzen, Erdalkalimetallsalzen, Zinksalzen oder Salzen mit einem organischen Amin vorliegen.

3. Verbindungen nach Anspruch 1 oder 2, dadurch gekennzeichnet, daß die niederen Alkylgruppen unter Methyl, Ethyl, Isopropyl, Butyl, t-Butyl, Hexyl, Nonyl und Dodecyl ausgewählt sind.

4. Verbindungen nach einem der vorhergehenden Ansprüche, dadurch gekennzeichnet, daß die geradkettigen oder verzweigten Alkylgruppen mit 1 bis 20 C-Atomen unter Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter 2-Hydroxyethyl, 2-Hydroxypropyl und 3-Hydroxypropyl ausgewählt sind.

6. Verbindungen nach einem der vorhergehenden Ansprüche, dadurch gekennzeichnet, daß die Polyhydroxyalkylgruppen unter 2,3-Dihydroxypropyl, 2,3,4-Trihydroxybutyl, 2,3,4,5-Tetrahydroxypentyl und dem Pentaerythrit-Rest ausgewählt sind.

7. Verbindungen nach einem der vorhergehenden Ansprüche, dadurch gekennzeichnet, daß die Arylgruppe eine Phenylgruppe ist, die ggf. mit einem oder mehreren Halogenatomen, einer oder mehreren Hydroxygruppen oder einer oder mehreren Nitrofunktionen substituiert ist.

8. Verbindungen nach einem der vorhergehenden Ansprüche, dadurch gekennzeichnet, daß die Aralkylgruppen unter Benzyl und Phenetyl ausgewählt sind, die ggf. mit einem oder mehreren Halogenatomen, einer oder mehreren Hydroxygruppen oder einer oder mehreren Nitrofunktionen substituiert sind.

9. Verbindungen nach einem der vorhergehenden Ansprüche, dadurch gekennzeichnet, daß die Alkenylgruppen unter Gruppen ausgewählt sind, die 2 bis 5 C-Atome enthalten und eine oder mehrere ethylenische Doppelbindungen aufweisen, und insbesondere Allyl darstellen.

10. Verbindungen nach einem der vorhergehenden Ansprüche, dadurch gekennzeichnet, daß die Zuckerreste unter Resten von Glucose, Galactose oder Mannose oder von Glucuronsäure ausgewählt sind.

11. Verbindungen nach einem der vorhergehenden Ansprüche, dadurch gekennzeichnet, daß die Aminosäurereste unter Resten ausgewählt sind, die von Lysin, Glycin oder Asparaginsäure abgeleitet sind.

12. Verbindungen nach einem der vorhergehenden Ansprüche, dadurch gekennzeichnet, daß die Peptidreste unter Resten von Dipeptiden oder von Tripeptiden ausgewählt sind.

13. Verbindungen nach einem der vorhergehenden Ansprüche, dadurch gekennzeichnet, daß die heterocyclischen Gruppen unter Piperidino, Morpholino, Pyrrolidino und Piperazino ausgewählt sind, die ggf. in 4-Stellung mit einer C₁₋₆-Alkylgruppe oder einer Mono- oder Polyhydroxyalkylgruppe substituiert sind.

14. Verbindungen nach einem der vorhergehenden Ansprüche, dadurch gekennzeichnet, daß die Halogenatome unter Fluor, Chlor und Brom ausgewählt sind.

15. Verbindungen nach Anspruch 1, dadurch gekennzeichnet, daß sie folgende Verbindungen darstellen, die allein oder in Form von Gemischen vorliegen:
6-(5,6,7,8-Tetrahydro-5,5,8,8-tetramethyl-2-naphthyloxy)-naphthoesäure,
4-(5,6,7,8-Tetrahydro-5,5,8,8-tetramethyl-2-naphthyloxy)-zimtsäure,
β-Methyl-(4-(5,6,7,8-tetrahydro-5,5,8,8-tetramethyl-2-naphthyloxy)-zimtsäure,
4-(5,6,7,8-Tetrahydro-5,5,8,8-tetramethyl-2-naphthyloxy)-phenylpropiolsäure,
6-(3,5,5,8,8-pentamethyl-5,6,7,8-tetrahydro-2-naphthyloxy)-naphthoesäure,
6-(5,6,7,8-Tetrahydro-5,5,8,8-tetramethyl-2-naphthylthio)-naphthoesäure,
6-(5,6,7,8-Tetrahydro-5,5,8,8-tetramethyl-2-naphthylsulfinyl)-naphthoesäure,
6-(5,6,7,8-Tetrahydro-5,5,8,8-tetramethyl-2-naphthylsulfonyl)-naphthoesäure,
6-(5,6,7,8-Tetrahydro-5,5,8,8-tetramethyl-2-naphthylamino)-naphthoesäure,
α-Hydroxy-4-(5,6,7,8-tetrahydro-5,5,8,8-tetramethyl-2-naphthyloxy)-zimtsäure,
α-Acetamido-4-(5,6,7,8-tetrahydro-5,5,8,8-tetxamethyl-2-naphthyloxy)-zimtsäure,
6-(3,5,5,8,8-Pentamethyl-5,6,7,8-tetrahydro-2-naphthylthio)-naphthoesäure,
6-(3,5,5,8,8-Pentamethyl-5,6,7,8-tetrahydro-2-naphthylsulfonyl)-naphthoesäure,
6-(3-Brom-5,6,7,8-Tetrahydro-5,5,8,8-tetramethyl-2-naphthyloxy)-naphthoesäure,
6-(5,6,7,8-Tetrahydro-5,5,8,8-tetramethyl-2-naphthyloxy)-naphthalinmethanol,
N-Ethyl-6-(5,6,7,8-tetrahydro-5,5,8,8-tetramethyl-2-naphthyloxy)-naphthalincarboxamid,
6-(5,6,7,8-Tetrahydro-5,5,8,8-tetramethyl-2-naphthyloxy)-naphthalincarboxamid,
6-(5,6,7,8-Tetrahydro-5,5,8,8-tetramethyl-2-naphthyloxy)-naphthoesäuremorpholid
und
6-(5,6,7,8-Tetrahydro-5,5,8,8-tetramethyl-2-naphthyloxy)-naphthalincarboxaldehyd.

16. Verbindungen nach Anspruch 1, dadurch gekennzeichnet, daß sie mindestens eine der nachstehenden Eigenschaften aufweisen:
- R₁ ist eine Gruppe -CO-R₁₀,
- X bedeutet -O-, -S- oder -NR₁₂-,
- R₂ ist ein Wasserstoffatom oder eine niedere Alkylgruppe,
- R₂ und R₄ bilden mit dem benachbarten Benzolring einen Naphthalinring,
- R₂ und R₃ bilden mit der ethylenischen Doppelbindung eine Dreifachbindung.

17. Verbindungen nach einem der vorhergehenden Ansprüche zur Verwendung als Arzneimittel.

18. Verbindungen nach Anspruch 17 zur Verwendung als Arzneimittel zur Behandlung von dermatologischen, rheumatischen, respiratorischen, kardiovaskulären und ophthalmologischen Erkrankungen.

19. Verwendung von einer oder mehreren Verbindungen, die in den Ansprüchen 1 bis 16 definiert sind, zur Herstellung eines Arzneimittels zur Behandlung von dermatologischen, rheumatischen, respiratorischen, kardiovaskulären und ophthalmologischen Erkrankungen.

20. Pharmazeutische Zusammensetzung, dadurch gekennzeichnet, daß sie in einem pharmazeutisch geeigneten Träger eine oder mehrere Verbindungen nach einem der Ansprüche 1 bis 16 enthält.

21. Zusammensetzung nach Anspruch 20, dadurch gekennzeichnet, daß die Konzentration an einer oder mehreren Verbindungen nach einem der Ansprüche 1 bis 16 im Bereich von 0,001 bis 5 Gew.-%, bezogen auf das Gesamtgewicht der Zusammensetzung, liegt.

22. Kosmetische Zusammensetzung, dadurch gekennzeichnet, daß sie in einem kosmetisch geeigneten Träger eine oder mehrere Verbindungen nach einem der Ansprüche 1 bis 16 enthält.

23. Zusammensetzung nach Anspruch 22, dadurch gekennzeichnet, daß die Konzentration an einer oder mehreren Verbindungen nach einem der Ansprüche 1 bis 16 im Bereich von 0,001 bis 3 Gew.-%, bezogen auf das Gesamtgewicht der Zusammensetzung, liegt.

24. Verwendung einer kosmetischen Zusammensetzung nach Anspruch 22 oder 23 zur Körperhygiene oder Haarhygiene.
